(19) Europäisches Patentamt
European Patent Office
Office européen des brevets

(11) EP 0 494 734 B1

(12) **EUROPEAN PATENT SPECIFICATION**

(45) Date of publication and mention
of the grant of the patent:
**05.08.1998 Bulletin 1998/32**

(51) Int Cl.$^6$: **G01N 21/35**, G01N 33/28,
G01N 21/31

(21) Application number: **92300046.7**

(22) Date of filing: **03.01.1992**

(54) **A method and apparatus for determining the composition of a fuel mixture**

Verfahren und Gerät zur Bestimmung der Zusammensetzung einer Brennstoffmischung

Méthode et appareil pour déterminer la composition d'un mélange de carburants

(84) Designated Contracting States:
**DE FR GB**

(30) Priority: **07.01.1991 US 637738**

(43) Date of publication of application:
**15.07.1992 Bulletin 1992/29**

(73) Proprietors:
• **FORD MOTOR COMPANY LIMITED**
**Brentwood, Essex CM13 3BW (GB)**
Designated Contracting States:
**GB**
• **FORD-WERKE AKTIENGESELLSCHAFT**
**50725 Köln (DE)**
Designated Contracting States:
**DE**
• **FORD FRANCE S. A.**
**92506 Rueil-Malmaison Cédex (FR)**
Designated Contracting States:
**FR**

(72) Inventors:
• **Carduner, Keith R.**
**Dearborn, Michigan 48128 (US)**
• **Colvin, Alex D.**
**Oak Park, Michigan 48237 (US)**
• **Marano, Richard S.**
**Dearborn, Michigan 48126 (US)**
• **Renny, David G.**
**Milford, Michigan 48042 (US)**

(74) Representative: **Messulam, Alec Moses et al**
**A. Messulam & Co.**
**24 Broadway**
**Leigh-on-Sea Essex SS9 1BN (GB)**

(56) References cited:
EP-A- 171 300          EP-A- 346 609
WO-A-90/03565          US-A- 4 594 968
US-A- 4 749 274

## Description

The present invention relates to a method and apparatus for determining the composition of a fuel mixture.

It is useful for an engine to operate on a variety of fuels or fuel mixtures. Such fuels include gasoline with varying levels of aliphatic, olefinic, and aromatic components and alcohol such as methanol, ethanol, propanol, etc. The percentage of the alcohol component has the greatest effect on the ability of the engine to efficiently burn the fuel mixture and thus a method to determine the weight percentage of alcohol in the fuel is required for a vehicle running on fuel of varying alcohol level. A variety of methods and devices exist for measuring the alcohol content of a gasoline-alcohol fuel mixture. Each of these techniques can be made into a device that is mountable in the fuel system of a motor vehicle. These techniques include refractive index, capacitance, and near infrared absorbance.

An example of a device that measures the index of light refraction of a fuel is disclosed in U.S. Patent 4,438,749 issued to Schwippert, March 27, 1984. Schwippert teaches passing a beam of light through a fuel mixture and measuring the index of light refrection based on a critical-angle measurement along with a temperature sensing element for correcting the measured index of refraction in accordance with changes in the temperature of the fuel supply. Problems with fricative index include inaccurate measurements due to emulsification of the alcohol in the fuel, changes in refractive index of the fuel mixture due to other impurities in the fuel mixture and changes in refractive index at a constant alcohol fraction due to variations i the constituents of the gasoline component. Gasoline is a generic term for a complex mixture of at least three classes of hydrocarbons distributed over hundreds of specific compounds. Each of these components has a unique refractive index and thus the refractive index of "gasoline" can vary substantially.

Capacitive measuring devices such as the device in U.S. Patent 4,915,084 issued April 10, 1990 to Gonze teaches a dielectric property of the fuel mixture to determine the ratio of alcohol to gasoline. Dielectric measurements are susceptible to additives that lower the electrical resistance of the fuel. Changes in resistance that result from low levels of these impurities significantly change the calibration of the dielectric device. In addition, dielectric devices may be very sensitive to corrosive fuel additives or properties and may also be sensitive to spurious electrical signals in an engine compartment.

Near infrared absorbance has been used to determine the concentration of alcohol in a mixture. U.S. Patent 3,562,524, issued February 9, 1971 to Moore teaches the measurement of ethyl alcohol in alveolar air. This is a device for measuring alcohol vapour and is primarily related to breathalyser devices. Devices which teach the near infrared measurement of alcohol in a fuel include U.S. Patent 4,371,785, issued February 1, 1983 to Pedersen, and U.S. Patent 4,594,968, issued June 17, 1986 to Degobert, et al. Both Degobert, et al. and Pedersen teach a device having a sample chamber and a reference chamber. The reference chamber contains a known purity of alcohol. A light beam is split into two arms and each arm is passed through either the sample chamber and the reference chamber. The absorbance of both the sample and reference chambers are used to determine the proportion of alcohol in a fuel mixture. Neither Degobert et al. or Pedersen account for the emulsification of alcohol in gasoline at low temperatures. This emulsification reduces the accuracy of measurements made using these devices. Also, the use of a reference chamber significantly increases the cost and complexity of a sensor for use in automotive applications.

A commercially practicable sensor for determining the constituent content of an alcohol/gasoline mixture must be both relatively inexpensive to manufacture and reliable for the life of the automobile. In particular, when the sensor is used to determine the concentration of methanol in a fuel mixture, the sensor must take into account the variability in aromatic and aliphatic content of commercial fuels, the insolubility of methanol in gasoline at low temperatures and in the presence of water and the variation in the density of methanol as a function of temperature. Finally, the fuel mixture analysis should be performed on the mixture in the fuel line as close as possible to the engine to avoid false readings due to mixing inhomogeneity in the fuel tank.

EP-A-0,171,300 discloses a method of determining emulsification in a liquid mixture.

WO-A-90 03565 discloses a method and an apparatus for measuring alcohol concentration in an alcohol-gasoline mixture.

It is an object of the present invention to provide a low cost reliable method and apparatus for precisely measuring the percentage of chemical components of a fuel mixture over a wide range of temperatures corresponding to typical conditions for a motor vehicle

According to the invention there is provided a method for determining the proportion of a fuel in a fuel mixture comprising the steps of, emitting a light beam through said fuel mixture at two or more wave lengths, measuring the relative absorbance of said fuel mixture at each said wave length, measuring the temperature of said fuel mixture, and analysing said relative absorbance and temperature to determine the proportion of said fuel in said fuel mixture.

Further according to the invention there is provided an apparatus for determining the proportion of fuel in a fuel mixture, comprising a cell containing said mixture, a light source emitting light to said mixture including at least a first and a second wavelength, means for measuring the relative absorbance of said mixture at said first and second wavelength, means for measuring the temperature of said mixture, and means for analysing said relative absorbance and temperature to determine the proportion(s) of said fuel in said mixture.

At least two NIR wavelengths are used of light to measure a fuel component's near infrared absorbance peak wavelength and a reference wavelength.

The invention is primarily adapted to measure the amount of methanol in a mixture of gasoline and methanol. However, the device also measures the amount of ethanol in gasoline or a combination of methanol and ethanol in gasoline. other compounds having OH groups such as higher alcohols and water may also be measured using the methods and apparatus's taught by this disclosure. The same design can be extended to measure other non-OH containing components of fuel such as the aromatic component by changing the wavelengths of light that are monitored.

A wavelength of light is selected which corresponds to the peak absorbance of the component to be measured. Selection is made by reference to standard texts on near infrared spectra of compounds or by acquisition of a near infrared spectrum on a commercially available spectrometer. A typical commercial device is the Bran Luebbe Infralyzer 500, manufactured by Bran Luebbe, Elmsford, NY. For methanol, the peak absorbance is approximately 1580 nm (nanometers). A reference wavelength which is both in the vicinity of the peak wavelength and which is weakly absorbed by the fuel mixture is selected. Both the peak and reference wavelengths are passed through a cell having a gap of a fixed size. Fuel passes through the cell and between the gap. The cell is located as near as practical to the engine fuel rail to provide a near instantaneous measure of the fuel being burned by the engine.

One embodiment of the invention uses fibre-optic cables supplying and returning the peak and reference wavelengths. This embodiment permits the cell to be located in the vicinity of the engine and the electronics supplying and measuring the wavelengths to be at a remote location.

An alternative and preferred embodiment of the invention places both a light source and detector means within the cell. The space between the light source and the detector means comprises the gap through which the fuel mixture passes.

With both embodiments, the peak and reference wavelengths are used to determine the relative absorbance of a fuel mixture. This relative absorbance measurement is important because the absorbance of a fuel mixture varies greatly with temperature and the effects of emulsification. Emulsification produces a light scattering effect that appears to the electronic circuit as an increase in the absorbance of light by the fuel mixture. By using two frequencies of light, the relative absorbance between the peak and reference wavelengths remain proportional to the amount of the fuel component in the fuel mixture since the effect of emulsification is to produce almost exactly the same amount of light scattering at the two wavelengths. The effect of temperature is to change the density of the fuel and thus the apparent methanol concentration will be seen to change even though its relative fraction of the fuel mixture has not changed. The effect of fuel temperature on the sensor read-out is compensated as part of the invention. By correcting for the effects of temperature and emulsification on the sensing of the fuel mixture, an accurate measurement of a fuel component in a fuel mixture can be determined over a wide range of conditions.

The invention uses low cost components such as automotive light bulbs and relatively inexpensive photo detectors. A novel circuit made from inexpensive operational amplifiers compensates for the effect of ambient temperature on the electronics of the circuit itself and thus on output of the electronic circuit.

The output of a fuel sensor made from the teachings of this invention is provided to an electronic engine control computer. Various operating parameters such as air-to-fuel mixture, spark advance, exhaust gas recirculation as well as other parameters such as valve timing may be varied to provide optimum parameters for the fuel mixture entering a cylinder. By placing a fuel sensor as near as possible to the engine fuel rail, the precise composition of the fuel can be calculated just prior to injection into the cylinder without the need for time-delayed circuitry.

The invention will now be described further, by way of example, with reference to the accompanying drawings, in which :

Figures 1a and 1b are graphs of absorbance vs. wavelength for gasoline and methanol, respectively.

Figure 2 is a graph of absorbance vs. wavelength for mixtures of gasoline and methanol.

Figure 3 is a graph showing the effect on absorbance by emulsification of a methanol-gasoline mixture due to water.

Figure 4 is a graph showing the effect on absorbance by emulsification of a methanol-gasoline mixture due to temperature.

Figure 5 is a schematic of a methanol sensor attached to an engine.

Figure 6 is a schematic representation of the control module shown in Figure 5.

Figure 7A and 7B are graphs of the output of the control module for M20 and M10 respectively at 25°C and -10IC.

Figure 8 is a detailed circuit diagram of the control module shown in Figure 5.

Figure 9 is a schematic of an alternative embodiment of a methanol sensor attached to an engine.

Figure 10 is a schematic representation of an alternative embodiment of the control module shown in Figure 8.

Figure 11 is a detailed circuit diagram of the control module shown in Figure 8.

Figure 12 is a graph of absorbance vs. wavelength for ethanol, methanol and water.

Figure 13 is a graph of absorbance vs. wavelength for a series of fuels having different aromatic component percentages.

Figure 14 is a circuit diagram of an emulsification sensor.

EP 0 494 734 B1

The present invention is capable of measuring the methanol concentration in an automotive fuel mixture based on the absorbance of light at specific wavelengths in the near infrared region. Minor modifications involving a change of the wavelengths of light that are used will make the device sensitive to the aromatic components or other components of a typical fuel. The near infrared region comprises the wavelengths between 650 - 2500 nm. Optical absorbencies are composed of overtones and combination bands of either C-H, N-H, or O-H stretching fundamentals found in the mid-infrared region between 3000 - 4000 nm.

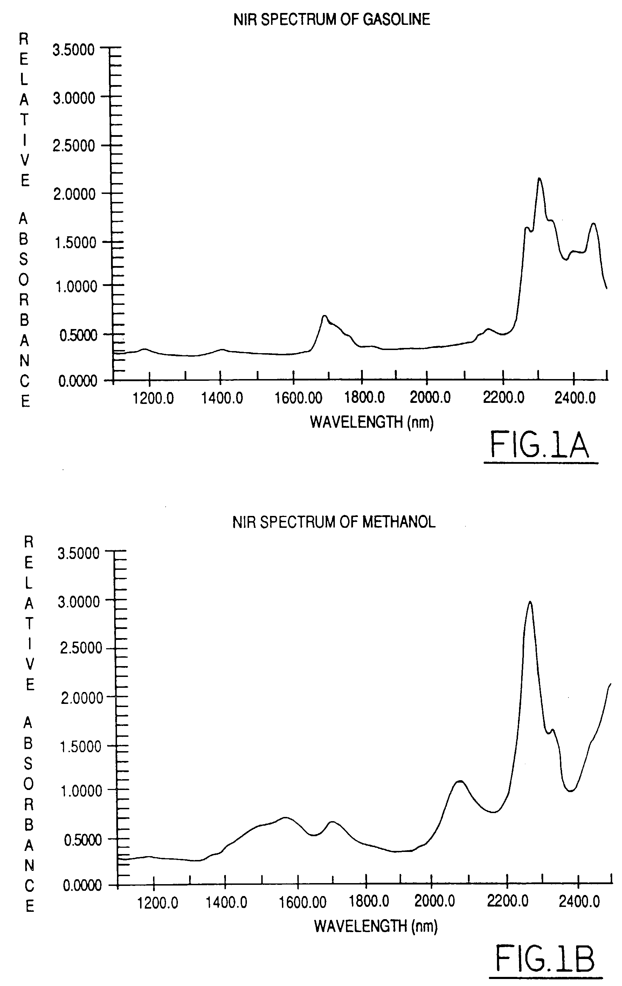

Figure 1a shows a plot of absorbance for a typical commercial grade gasoline between the wavelengths 1100 and 2500 nm. Figure 1b shows a plot of 100% methanol over the same wavelength range. Absorbance as used herein is defined as follows:

$$Abs = -\log(I_{in}/I_{out}),$$

where $I_{in}$ is the total light available at a specific wavelength and $I_{out}$ is the light that is transmitted through the sample. $I_{in}$ may be measured in a number of ways including directly measuring the output of the beam before it enters the sample. $I_{out}$ is the amount of light that reaches a detector after having passed through the sample. The difference between $I_{in}$ and $I_{out}$ is primarily due to light being attenuated by either absorbance or scattering of the sample.

The relative absorbance ($ABS_{rel}$) is defined as the difference in absorbance between two wavelengths of light. It is calculated as follows:

$$ABS_{rel} = ABS1 - ABS2$$

$$= -\log(I^1_{in}/I^1_{out}) + \log(I^2_{in}/I^2_{out})$$

$$= -\log(I^2_{out}/I^1_{out}) + \log(I^2_{in}/I^1_{in}).$$

Since the intensity of the available light, $I_{in}$, remains constant, the term $\log(I^2_{in}/I^1_{in})$ is either zero or constant. Therefore, an absorbance peak can be measured by determining the absorbance at the peak wavelength and at a suitable reference wavelength without the need for measuring the intensity of the incoming light.

Standard references on near infrared spectra such as M. Avram, "Infrared Spectroscopy" (Wiley, New York, 1972), incorporated herein by reference, explain that the overtone absorbencies related to the C-H stretching motions from aliphatic components of gasoline make up the predominant features lying between 2250 and 2500 nm in Figure la as well as the smaller peaks at 1190 and 1400 nm. The peaks lying between 1700 and 1800 nm are also derived from the aliphatic hydrocarbon components of gasoline. Aromatic components of gasoline produce their own distinct features of 2150 and 1150 nm. Because of the distinct spectral features originating from the aromatic and aliphatic components of gasoline, it is possible to determine the proportion of aliphatic to aromatic components of the fuel.

The spectrum of methanol is shown in Figure 1b. The features that relate to C-H stretching motion of the methyl group include the peaks lying between 2000 and 2500 nm, 1700 and 1800 nm, and the small peak at 1200 nm. Methanol also includes an additional feature originating from the O-H stretching motion observed between 1400 and 1650 nm for which there is no corresponding feature in unoxygenated gasoline. This feature allows for the facile measurement of the methanol component in a gasoline-methanol mixture.

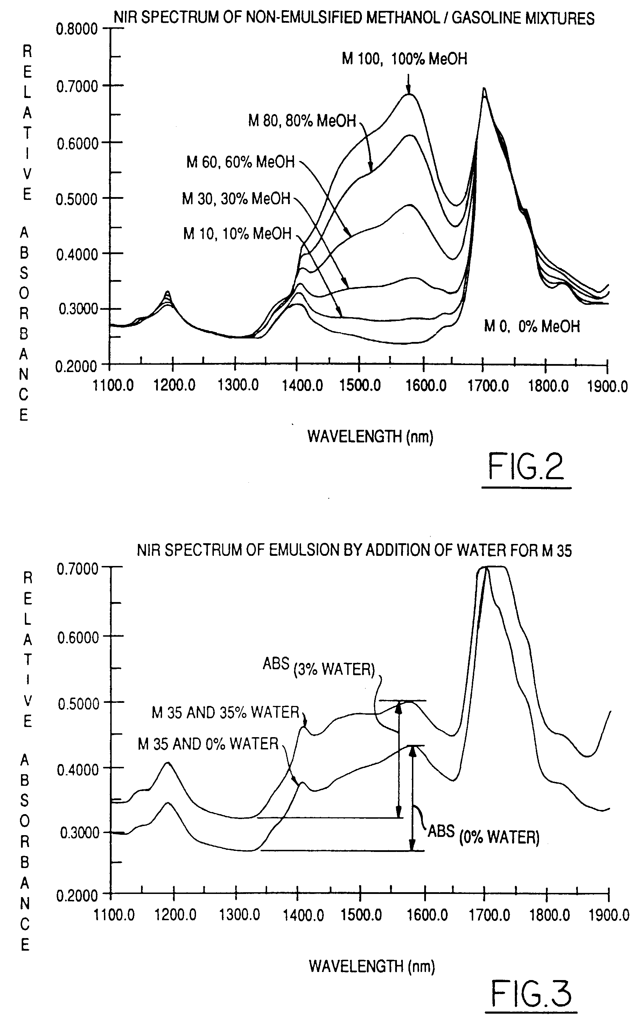

Shown in Figure 2 is a composite graph of the relative absorbance verses wavelengths for non-emulsified methanol- gasoline mixtures. M0 corresponds to pure gasoline containing 0% methanol. M10 corresponds to a methanol-gasoline mixture containing 10% by volume methanol. M100 corresponds to pure methanol. From viewing Figure 2, it is apparent that the absorbance in the vicinity of 1580 nm corresponds essentially to the concentration of methanol. In particular, the absorbance of the fuel mixture at 1580 nm relates approximately linearly to the concentration of methanol. However, despite the apparent linearity of the relationship of absorbance to concentration suggested by this graph, simply measuring the peak absorbance at 1580 nm does not always provide the proportion of methanol in a methanol-gasoline mixture due to the effects of temperature, emulsification and impurities such as water.

Mixtures of methanol and gasoline may emulsify under certain conditions, especially when the fuel is contaminated with a small percentage of water (as low as 0.25%). Even in the absence of water, the mixture may emulsify at temperatures below 25°C. In particular, emulsification in the absence of water is a function of the fuel temperature and the percentage of the aromatic component of the gasoline. Emulsification is herein defined as a reduction in the clarity of the fuel mixture arising from the immiscible nature of gasoline and methanol. Under certain conditions that relate to the percentage of aromatic component, temperature, or presence of water, the methanol and gasoline will physically separate into distinct phases and produce a mechanical mixture of small particles of the two fuels when they are being simultaneously pumped through a flowing system. Emulsified methanol-gasoline mixtures appear milky or cloudy.

4

Emulsification is most likely to occur for fuel mixtures between M20 to M60 and under temperatures that can be expected in the normal operation of an automobile. Emulsification is likely if the fuel is contaminated with water.

Other than water's effect on emulsification of the fuel mixture, it does not appear to significantly affect the NIR absorption spectra of a composition containing methanol and gasoline. The O-H bond of water has an absorbance peak of approximately 1440 nm and experimental combinations of up to 5% water in methanol-gasoline mixtures show only a minor absorbance in the vicinity of 1580 nm. This absorbance can be avoided by measuring the absorbance of methanol at 1600 nm. This shifts the absorbance far enough from the water peak at 1440 nm to avoid any effect from the water. Therefore, the presence of water in a non-emulsified methanol-gasoline mixture will not affect the absorbance in the vicinity of 1600 or 1300 nm. To precisely measure the concentration of water in a methanol-gasoline mixture, a third or different wavelength approximately in the vicinity of 1440 nm is necessary.

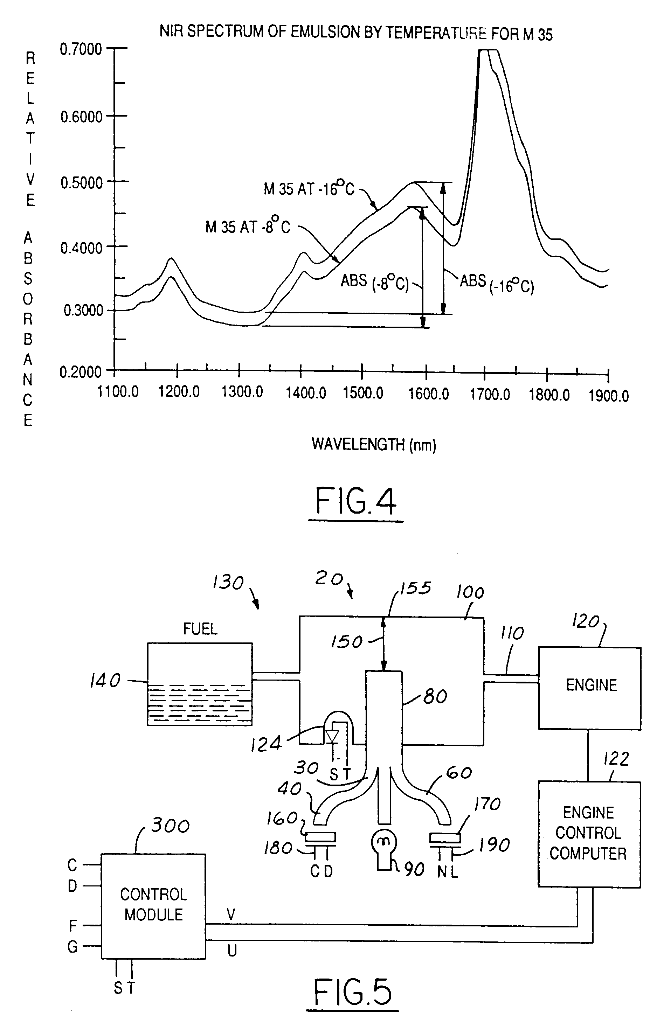

Although water does not directly contribute to absorbance at 1600 nm, it does contribute to phase separation of methanol-gasoline mixtures. The phase separation results in the emulsification of the mixture in flowing fuel. Emulsification causes the scattering of light. This light scattering causes the appearance of increased absorbance at both the 1300 and 1600 nm wavelengths although absorbance in the presence of emulsification does not increase. The effect of emulsification is to increase the apparent absorbance at both the 1600 and 1300 nm wavelengths, as is shown in Figure 3. Figure 3 is a plot of relative absorbance verses wavelength for M35. The bottom graph shows the non-emulsified fuel containing no water. The top graph shows the emulsification when 3% by volume water is added to M35 at room temperature. The baseline shift is a result of the light scattering effect of the emulsification. Some additional spectral intensity is observed in the top graph in the vicinity of 1440 nm which can be attributed to the absorption of the water molecules in the mixture. The light scattering due to the emulsification of the mixture affects both absorbencies at 1300 and 1600 nm. This result is an approximately 20% increase in the relative absorbance. If a device were properly calibrated to read the non-emulsified non-water containing M35 as 35% methanol by only reading the absorbance at 1600 nm, the emulsification due to 3% by volume water would increase this reading to approximately 55% methanol. Because the effect of emulsification is approximately the same at both 1300 and 1600 nm, the absorbance with water [ABS (3% water)] is approximately equal the absorbance without water [ABS (0% water)]. A device that determines the relative absorbance between 1300 and 1600 nm still gives a reading of approximately 35% methanol because the relative absorbencies is largely unaffected by the emulsification shift. A level of 3% by volume water is highly unlikely in commercial fuel mixtures and would likely approach 1% by volume in worse cases. However, a 1% water volume is sufficient to induce emulsification and light scattering caused thereby. Since the invention uses relative absorbance to determine the methanol content, the accuracy of measuring the methanol content of a fuel mixture containing water is relatively independent of the emulsification.

Like water, temperature also affects absorbance measurement of methanol-gasoline mixtures. Methanol- gasoline mixtures ranging from approximately M20 to M60 will readily emulsify below certain temperatures, even in the absence of water. Emulsification is more a problem with gasolines having low aromatic content. For these gasolines, emulsification temperatures have been reported as high as 20°C. Figure 4 shows the effect of emulsification on an M35 mixture between -8°C and -16°C. As in the case with the emulsification due to water, emulsification due to temperature increases the relative absorbance at the wavelengths 1600 and 1300 nm. In a sample of M35 containing no water, no emulsification was exhibited at -8°C. At -16°C, emulsification increased the absorbance by approximately 10%. This increase in absorbance would translate to a reading of 45% methanol rather than 35% methanol if only the absorbance at 1600 nm were monitored. Again using the relative absorbance between 1300 and 1600 nm, the difference between ABS (-16°C) and ABS (-8°C) is approximately 2%. This 2% margin of error is well within the accuracy necessary for use to control an automobile engine.

Another effect of temperature is related to the change in density of the methanol in the volume sampled in the cell. The volume of fuel flowing through the cell does not change since the sampling volume is defined by a cell having a fixed gap. Consequently, as the temperature is reduced, the density of the fuel increases. More methanol is confined within the gap and is available to absorb light at the peak wavelength. The opposite occurs as the temperature is increased above room temperature. When using only the two wavelengths of 1600 and 1300 nm, only the amount of methanol component is observed. The change in reading due to the apparent increase in methanol must be compensated for. Measuring the effects of temperature on a variety of methanol-gasoline mixtures, it has been determined that the affect of temperature is approximately linear for a constant percentage of methanol and proportional to the total amount of methanol in the mixture. Based on these tests, it has been determined that the compensation for temperature for a methanol-gasoline mixture may be approximated by the following equation:

$$PM_{comp} = \{1+.006(t-25)\}PM \qquad\qquad (eq.\ 2),$$

where PM is the percent methanol determined by the device without temperature compensation, t is the temperature of the fuel mixture, and $PM_{comp}$ is the temperature compensated methanol percentage. By applying this correction factor to the methanol percentage determined away from 25°C, the effect of temperature on the measurement of methanol can be corrected. The present invention uses this absorbance for a fuel mixture of methanol/gasoline and methanol/ethanol/gasoline.

A block diagram of a fuel sensor which utilises near infrared absorption of the fuel mixture is shown in Figure 5. Engine 120 is designed to operate on a mixture of fuels ranging from M0 to M85. Engine 120 can also operate on a gasoline/ethanol or gasoline/methanol/ethanol fuel mixture. Engine 120 is controlled by an electronic engine control computer 122. Engine 120 is connected to fuel sensor 20 through fuel rail 110. Sensor 20 comprises a trifurcated fibre-optic cable 30. Cable 30 contains a bundle of optical fibres that are sectioned into three arms 40, 50, 60 at one end and bundled together into a single arm at the opposite end 80.

Arm 50 carries a beam of light from light source 90 into in-line fuel cell 100. Fuel cell 100 is intended for insertion into the fuel line near to but before fuel rail 110 of engine 120. Insertion into the fuel rail is also possible but may require two sensors 20 because many fuel rails are typically split into two paths. Fuel cell 100 receives fuel from a fuel supply system 130 which comprises fuel tank 140, a fuel sender unit (not shown) and optionally a fuel pump (not shown). A beam of light having a wide spectrum of wavelengths but specifically including a peak absorbance wavelength of 1600 nm, and a reference wavelength of 1300 nm passes through arm 50 to end 80. The beam of light passes through gap 150 between end 80 and interior wall 160 of cell 100. The gap used in the present embodiment is approximately 1.5 mm. The beam of light is reflected by the mat surface (35 micron finish) of wall 160 and is collected by end 80. Total path length of light as it passes through the flowing fuel mixture is therefore approximately double the width of gap 150 - in this case 3 mm. Light not absorbed by the fuel (transmitted light) is passed through end 80 to arms 40 and 60. The transmitted light at arms 40 and 60 includes all wavelengths supplied by light source 90. An interference filter 160 filters all wavelengths except those at approximately 1600 nm corresponding to the peak absorbance wavelength. Interference filter 170 filters all the wavelengths other than those that are approximately 1300 nm corresponding to the reference wavelength. Detector 180 measures the intensity of the transmitted light corresponding to the peak absorbance wavelength while detector 190 measures the transmitted light corresponding to the reference wavelength. Detectors 180 and 190 can be any type of photo detector capable of measuring light in the peak and reference wavelengths, however, particularly preferred are detectors made of InGasAs manufactured by General Electric.

Fuel cell 100 also contains a glass package diode 124 whose electrical characteristics change as a function of temperature. Diode 124 is positioned in the fuel mixture flow path. Diode 124 can be any type diode generally sensitive to temperature, specifically preferred is diode model #1N914 manufactured by General Electric Corporation. Diode 124 has leads ST inputting to control module 300. Detector 180 has leads CD and detector 190 has leads NL supplying their output to module 300.

The choice of the optical path length corresponding to the distance of gap 150 is determined by the sensitivity of detectors 180 and 190 to absorbance by methanol and the amount of light lost due to emulsification. The sensitivity to the methanol absorbance in a fuel mixture increases with the path length at approximately a rate of 0.5 absorbance units for each millimetre of path length for an M100 sample. The reduction in light due to emulsification is approximately 0.2 absorbance units per millimetre or about .6 mm for the 3 mm path length. A path length of approximately 3 mm represents a good compromise between sensitivity and light reduction based on the current InGasAs photo detectors. Filter 160 was chosen to filter light centred at approximately 1600 nm rather than a methanol peak absorbance of 1580 nm to avoid the peak of the water absorption at approximately 1440 nm. Therefore, filter 160 monitors the absorbance at approximately 20 nm away from the true peak of the methanol absorbance to reduce any interference from any water in the methanol/gasoline mixture. The shift away from the true peak results in no more than a 5% reduction in the sensitivity of the device to the methanol absorbance.

InGaAs filters were chosen because they provide a flat response over temperatures in the range of -40 to 120°C at both 1600 and 1300 nm. If the response of the detectors changes over this temperature range, then the output of the detectors would have to be compensated for in module 300 or computer 210. Use of InGaAs detectors simplifies the electronics required for module 300.

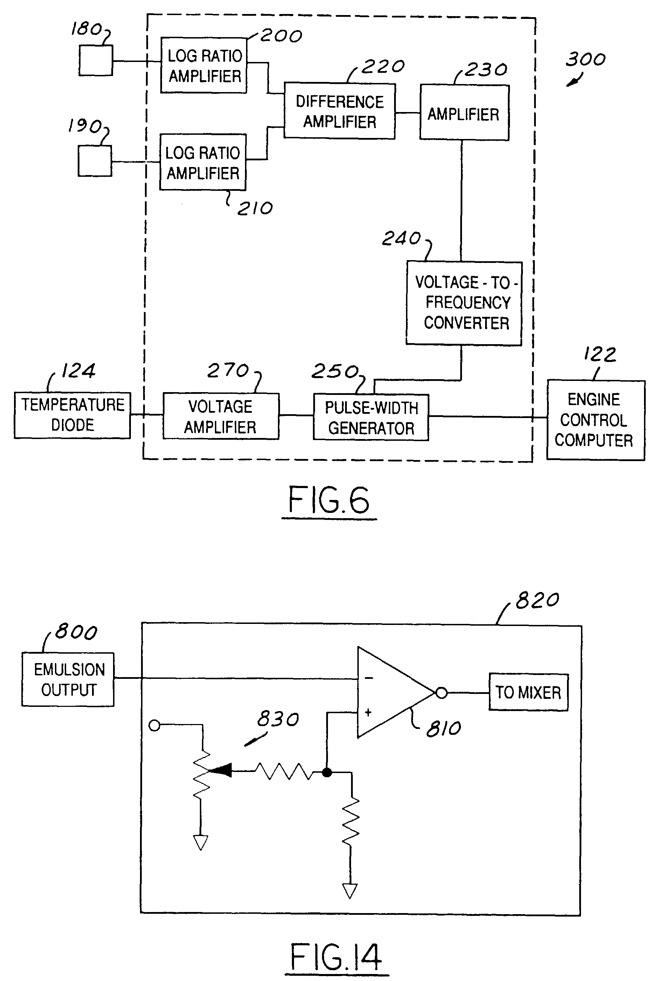

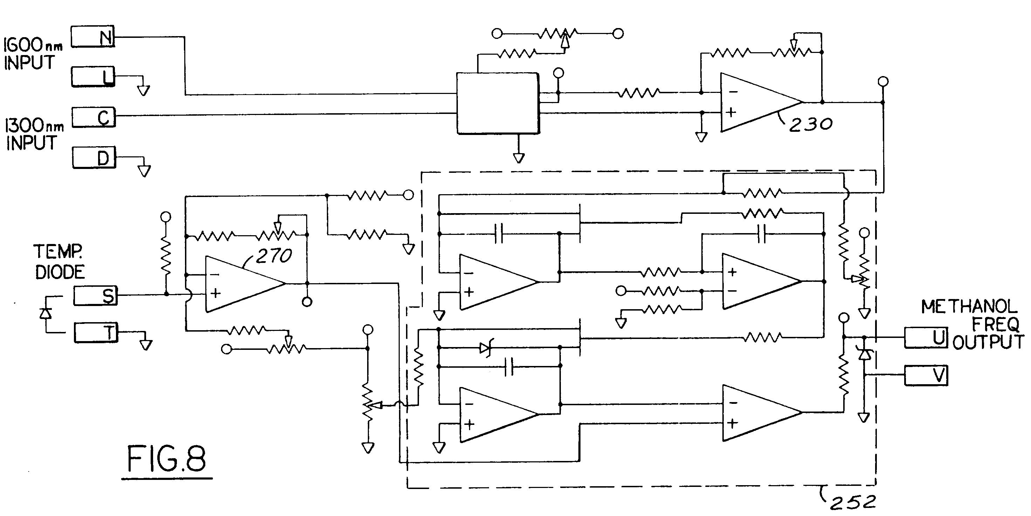

A block circuit diagram of module 300 is shown in Figures 6. A detail circuit diagram for module 300 is shown is Figure 8. Referring now to Figure 6, detectors 180 and 190 receive light from light source 90 (fibre optics and filters not shown in this block diagram). They produce a current proportional to the amount of light at either 1600 or 1300 nm that they respectively see. Their output goes to log amps 200 and 210 and then the difference of the log values is taken by difference amp 220. The output of the difference amp is the uncorrected absorbance difference between the two wavelengths. Amplifier 230 receives the signal from difference amp 220. The output of 230 is supplied to voltage-to-frequency converter 240. Converter 240 produces a square wave signal in a format acceptable for engine control computer 210. The square wave signal from converter 240 is supplied to pulse-width generator 250 which is used to control the width of the square wave pulses without affecting their frequency. Generator 250 receives a voltage from diode 124 and moderates the width of the square wave in proportion to the voltage drop across diode 124. The voltage drop in diode 124 is a linear function of the fuel temperature. The interface between diode 124 requires voltage amp 270. The affect of methanol and temperature are represented in the output wave of generator 250.

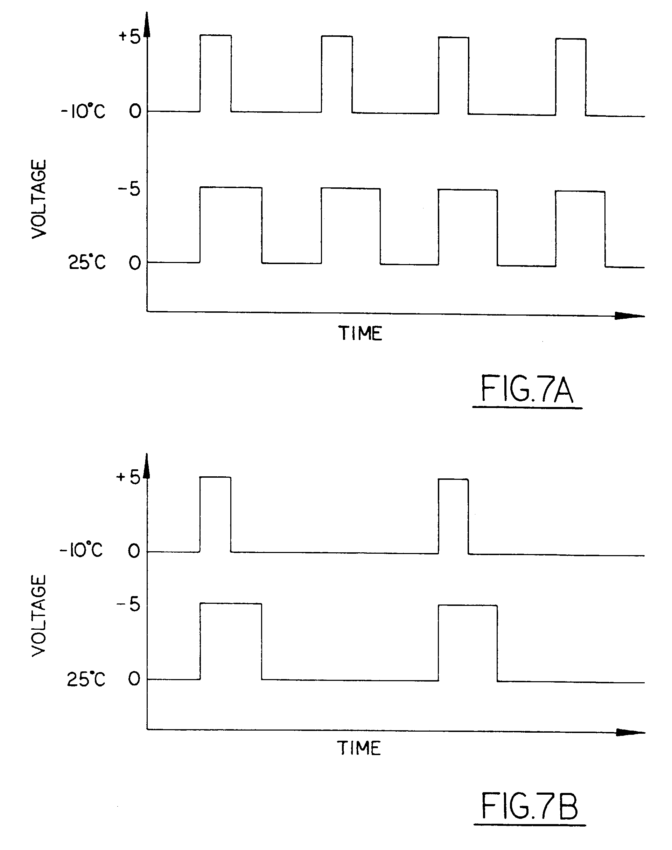

Shown in Figures 7A and 7B, are representative square wave outputs for a fuel mixture of M10 and M20 at -10°C and 25°C. Decreasing the temperature from 25°C to -10°C reduces the pulse width. Increasing the methanol concentration from M10 to M20 increases the pulse frequency. This output contains both the methanol concentration (uncorrected for fuel mixture temperature) and the fuel mixture temperature. The corrected methanol concentration may be determined by computer 122 using the output of generator 250 and equation 2.

With reference to the detailed electronic circuit of Figure 8, the components 200, 210, and 220 are combined within a package and commercially available as Log Ratio Amplifier AD757P manufactured by Analog Devices. Output amp 230 and diode buffer amp 270 are commercially available as amplifier OP07 manufactured by Precision Monolithics Incorporated. Voltage-to-frequency converter 240 and pulse- width generator 250 are represented on Figure 8 by dashed block labelled 252. These components are commercially available as LM324 manufactured by National Semiconductor.

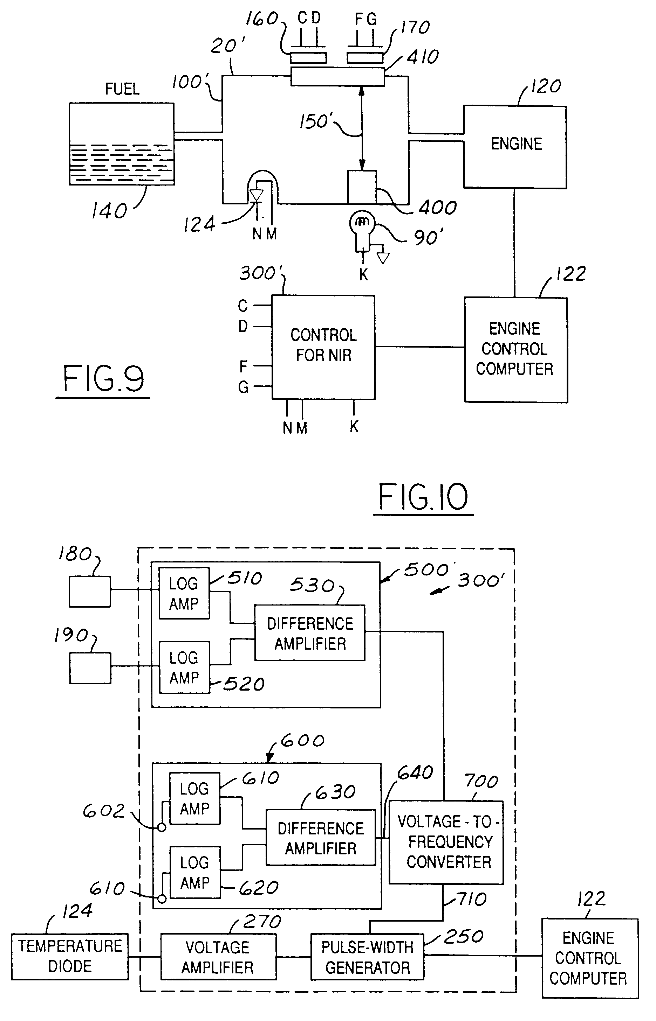

An alternative and more preferred embodiment of NIR sensor 20' is shown in Figure 9. Sensor 20' places light source 90' and detectors 180' and 190' adjacent the in-line fuel cell 100'. Placing the light source and the detectors adjacent cell 100' eliminates the need for fibre-optic cables and reduces the overall size and complexity of the sensor. Light source 90' can be achieved by either placing a bulb within cell 100' or by placing window 400 within fuel cell 20' through which a light beam shines. Detectors 180' and 190' can either be placed within the fuel flow path or behind window 410 through which a transmitted light passes. Light passes directly from window 400 to window 410. Because the light path is not reflected, the width of gap 150' is approximately twice that of gap 150 in Figure 5. This maintains a light path of approximately 3 mm.

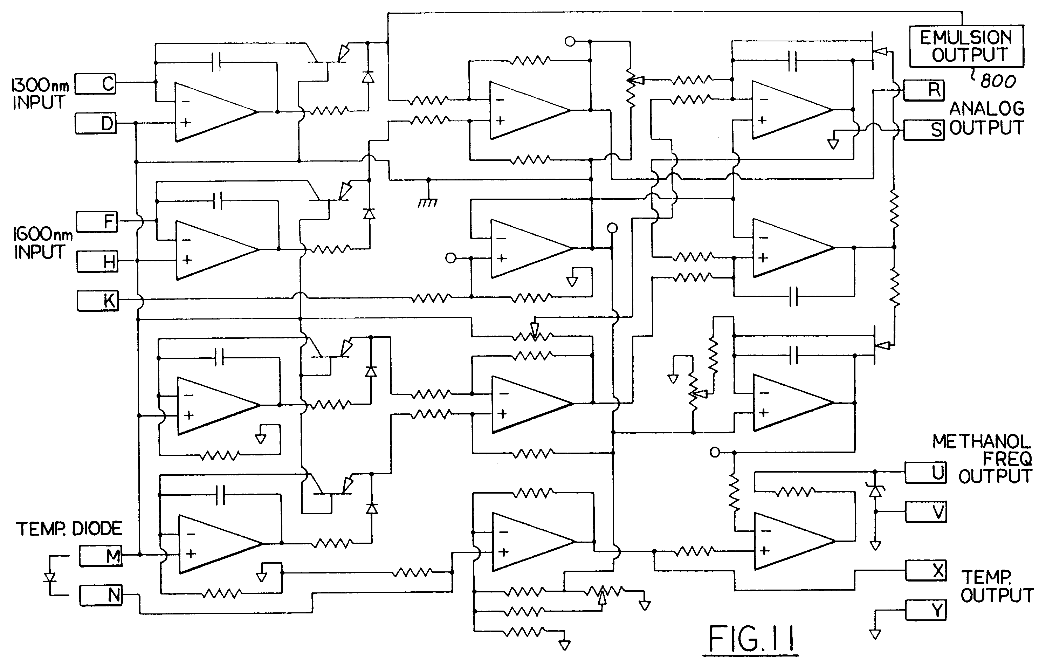

Control module 300' uses low cost log amps rather than the relatively expensive log ratio amplifiers 200,210 used in module 300. Shown in Figures 10 and 11 are a schematic and detailed circuit diagrams, respectively, of electronic module 300'. With reference to Figure 10, the output of detectors 180' and 190' are supplied to a primary comparator circuit 500. Comparator circuit 500 comprises two log amps 510 and 520 receiving the output of detectors 190' and 180' and supplying the log of the detector output to difference amplifier 530. Amplifier 530 supplies an absorbance difference voltage 540 to voltage-to-frequency converter 200. The output of log amps 510 and 520 and difference amp 530 is temperature dependent. To compensate for this temperature dependence, a temperature compensation circuit 600 comprising log amps 610 and 620 and difference amp 630 is used. The components 610, 620 and 630 are identical with the components 510, 520 and 530 respectively. A constant peak voltage 602 which simulates the output of detector 180' is supplied to log amp 610. A constant reference voltage 603 which simulates the output of detector 190', is supplied to log amp 620. A difference amplifier 630 compares the voltage from log amps 610 and 620 and provides a temperature compensating voltage 640 to a voltage-to-frequency converter 700.

Voltage-to-frequency converter 700 samples the compensating voltage 640. Compensating voltage 640 allows converter 700 to determine an ambient temperature compensation factor and correct absorbance voltage 540 by this factor. Converter 700 also produces a square wave output 710 which is corrected for the ambient temperature effect on components 510, 520 and 530. This ambient temperature correction differs from the fuel mixture temperature correction factor. Output 710 must still be corrected for the fuel mixture temperature.

As discussed in the embodiment shown in Figures 5 - 8, pulse width generator 250 provides an output square wave signal which contains both the methanol concentration (uncorrected for fuel mixture temperature) and the fuel mixture temperature. This output is supplied to an engine control computer 122.

Engine control computer 122, shown in Figure 9, uses equation 2 to correct the fuel mixture temperature's effect on the percentage of methanol. Engine control computer 122 can be used to optimise the operating parameters of an engine for the type of fuel being burned. An example of a control system for an engine using a fuel mixture of methanol and gasoline is shown and described in U.S. Patent 4,706,630, issued November 17, 1987 to Wineland, et al. incorporated herein by reference, U.S. Patent 4,706,629, issued November 17, 1987 to Wineland, et al. incorporated herein by reference, and U.S. Patent 4,703,732, issued November 3, 1987 to Wineland, et al. incorporated herein by reference.

Engine variable parameters include air-to-fuel ratio mixture, spark advance, exhaust gas recirculation, turbo boost, and variable valve timing as well as other parameters.

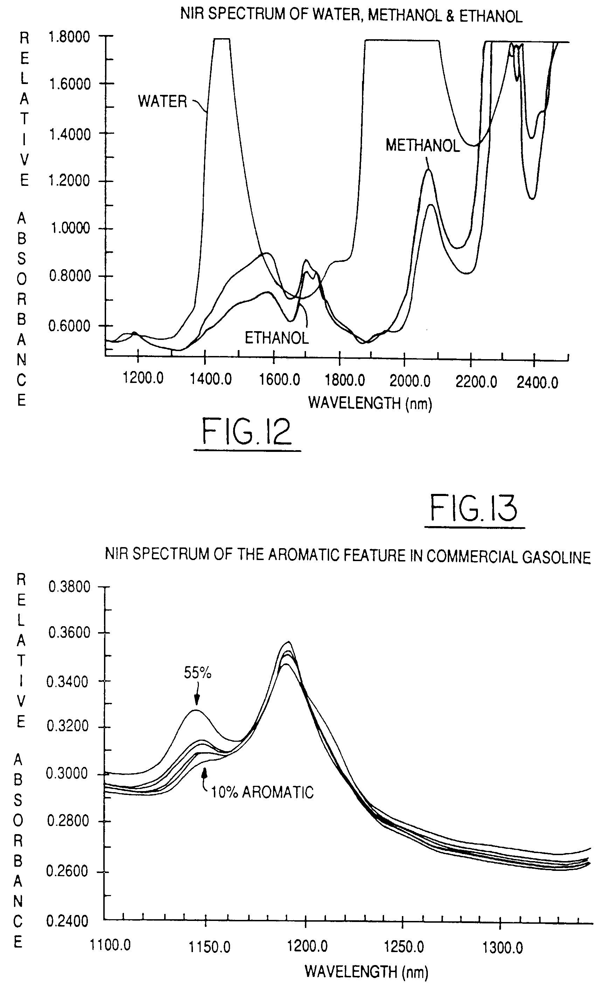

The invention as described is primarily directed to a sensor capable of determining the amount of methanol in a methanol-gasoline mixture. It is possible to use the fuel sensor of the disclosed embodiments in a vehicle burning a mixture of methanol, ethanol and gasoline. Ethanol also contains an O-H bond which exhibits a similar absorbance peak in the vicinity of 1600 nm. The spectra of methanol and ethanol are roughly comparable, as shown in Figure 12. Ethanol displays an absorbance response approximately 62% of that of methanol. Therefore, if pure ethanol were passed through a fuel sensor as described in Figures 5 and 9 which was calibrated for methanol, it would provide a response of approximately 62% and would assume the mixture was M62. If a sensor is calibrated for methanol at 1600 nm, it can be shown that the use of this sensor to determine the air-to-fuel ratio (afr) a fuel mixture containing up to 20% ethanol is less than 2%. To show this, the formula for the air-to-fuel ratio for a arbitrary mixture of methanol, ethanol, and gasoline is given by the following equation:

$$\text{afr}_{\text{mixture}} = \text{afr}_{\text{gasoline}}(1 - \text{PM}/100 - \text{PE}/100) + \text{afr}_{\text{EOH}}(\text{PE}/100) \qquad \text{(eq. 3)},$$

where:

$\text{afr}_{\text{mixture}}$ = Air-to-fuel ratio for methanol/ethanol/gasoline mixture,
$\text{afr}_{\text{gasoline}}$ = Air-to-fuel ratio for gasoline,
$\text{afr}_{\text{MeOH}}$ = Air-to-fuel ratio for methanol,
$\text{afr}_{\text{EOH}}$ = Air-to-fuel ratio for ethanol,
PM = Percent methanol by weight, and
PE = Percent ethanol by weight.

The afr for the various fuels may be analytically determined. The stoichiometric afr is exactly that amount of air required to completely burn the fuel to $CO_2$ and $H_2O$ without any residual fuel or oxygen. The formula for the stoichiometric afr for a fuel is:

$$\text{afr}_{\text{stoic}} = 29(1 + N/4 - P/2)/(.21(12 + N + 16P)) \qquad \text{(eq. 4)},$$

where:

N = number of hydrogen atoms, and
P = number of oxygen atoms.

Using equation 4, the stoichiometric afr's for gasoline, methanol and ethanol can be determined and are listed in Table I below:

TABLE I

| Fuel Type | N | P | afr |
|-----------|------|---|------|
| gasoline | 1.86 | 0 | 14.6 |
| methanol | 4 | 1 | 6.5 |
| ethanol | 6 | 1 | 9.01 |

Substituting these values into equation 3, the afr for a gasoline/methanol/ethanol mixture is represented by:

$$\text{afr} = 14.6(1 - \text{PM}/100 - \text{PE}/100) + 6.5(\text{PM}/100) + 9.01(\text{PE}/100) \qquad \text{(eq. 5)},$$

A fuel sensor calibrated for methanol can be used to provide information for both the PM and PE by the following equation:

$$S = \text{PM} + 0.62\,\text{PE} \qquad \text{(eq. 6)},$$

where:
S = sensor output of methanol/ethanol mixture and 0.62 is the percentage of absorbance of ethanol at 1600 nm.
A fuel sensor and engine having an air-to-fuel ratio properly calibrated for only methanol/gasoline fuel mixtures ($\text{afr}_{\text{cal}}$) would assume an air-to-fuel ratio of:

$$\text{afr}_{\text{cal}} = 14.6(1 - \text{PM}/100) + 6.5(\text{PM}) \qquad \text{(eq. 7)}$$

Substituting S for PM the equation becomes:

$$\text{afr}_{\text{cal}} = 14.6(1 - S/100) + 6.5(S) \qquad \text{(eq. 8)}$$

If the air-to-fuel ratios are calculated using both equations 5 and equations 8 above, then a comparison will show the error in using a fuel sensor calibrated only for methanol when used for determining afr's for mixtures of gasoline, ethanol, and methanol. Some representative errors are tabulated in Table II below:

TABLE II

| NIR Sensor afr Measurement Errors For Ethanol and Gasoline | | | |
|---|---|---|---|
| %MeOH | %EtOH | afr | %afr error |
| 0 | 20 | 13.482 | .8 |
| 20 | 20 | 11.856 | .9 |
| 40 | 20 | 10.230 | 1.1 |
| 60 | 20 | 8.604 | 1.3 |
| 80 | 20 | 6.978 | 1.6 |

While the invention as disclosed has been primarily described as a methanol sensor, the same concepts as presented in the embodiments can be used to determine other components of fuels including the amount of aromatic and aliphatic components. The aromatic component of fuel can be determined by monitoring the aromatic absorbance at 1148 nm as the absorbance peak and again using 1300 nm as the reference wavelength. Figure 13 shows that the peak at 1148 nm changes with the level of aromatics in commercial fuels and that no absorbance occurs at 1300 nm. A simple change of the wavelength in the device presented in this invention would make the sensor into an aromatics sensor. Alternatively, the device could monitor three or more wavelengths simultaneously and in such a fashion measure a number of fuel components.

The invention as described is related to a method and apparatus of determining the percentage of alcohol in gasoline even in the presence of emulsification. The invention also permits the detection of emulsification in a fuel mixture. This capability is useful to operate an additional device to reduce the effects of emulsification on an engine. These devices include various types of mixers that reduce the size of the emulsified particles. Continuous operation of these emulsion reducing devices may be undesirable from an energy, noise or efficiency standpoint. A separate emulsion sensor that signals the operation of these devices permits their operation only in the presence of emulsion.

An emulsion sensor of this type can be made separate from the fuel sensor using the absorbance at only one wavelength such as 1300 nm. Alternatively, the emulsion sensor can be integrated with the fuel sensor by using the absorbance of the sensor at the reference wavelength.

Figure 14 shows a detail circuit diagram for an emulsion sensor. It receives its' input from the emulsion output labelled 800 in Figure 11. This emulsion output 800 is used by emulsion sensor 820 in Figure 14. Emulsion sensor 820 contains a comparator 810 and variable resistor 830.

The shift due to the emulsion appears to be a constant amount of light reduction and thus the output of the 1300 nm detector (which is not affected by the absorbance by the methanol peak) can be used as an "on/off" indicator of emulsion. Comparator 810 compares the output of the 1300 nm detector against a predetermined voltage level. If the detector output exceeds the comparison level, then a non-emulsification condition exists, and the output of comparator 810 is -5V. If emulsification exists, the output of the 1300 nm detector drops below the comparison voltage level and the output of comparator 810 goes to +5V. This change in voltage may be used to signal another device, such as a mixture, of the presence of emulsion. Variable resistor 830 can be used to select the threshold level of emulsion before sensor 820 signals the presence of emulsion.

## Claims

1. A method for determining the proportion of a fuel in a fuel mixture over a wide range of temperatures comprising the steps of, emitting a light beam through said fuel mixture at two or more wavelengths, measuring the relative absorbance of said fuel mixture at each said wavelength, measuring the temperature of said fuel mixture the means used for measuring the temperature being distinct from the means used for measuring the relative absorbance, and analysing said relative absorbance and temperature to determine the proportion of said fuel in said fuel mixture.

2. A method as claimed in claim 1, wherein said wavelengths comprise a reference wavelength and a peak wavelength, said reference wavelength being selected to be weakly absorbed by said mixture and said peak wavelength being selected to be strongly absorbed by said mixture.

3. A method as claimed in any one of Claims 1 to 2, further comprising placing said mixture in a cell having a gap, before the step of emitting said light beam.

4. A method as claimed in Claim 3, wherein said cell has a fixed gap sized to provide nearly linear absorbance.

5. A method as claimed in Claim 4, further comprising passing said mixture through said cell and measuring the absorbance of said mixture at said gap.

6. A method as claimed in claim 2, wherein said peak wavelength is between 1400 and 1650 nm and said reference wavelength is between 1100 and 1350 nm.

7. An apparatus for determining the proportion of fuel in a fuel mixture over a wide range of temperatures, comprising a cell (100) containing said mixture, a light source (90) emitting light to said mixture including at least a first and a second wavelength, means (180, 190) for measuring the relative absorbance of said mixture at said first and second wavelength, means (124) for measuring the temperature of said mixture the means (124) for measuring the temperature being distinct from the means (180, 190) for measuring the relative absorbance, and means for analysing said relative absorbance and temperature to determine the proportion(s) of said fuel in said mixture.

8. An apparatus as claimed in claim 7, wherein said cell further comprises a gap and said relative absorbance is measured at said gap.

9. An apparatus as claimed in claim 8, wherein said cell further comprises a light source for supplying said wavelengths and a detector for measuring said wavelengths, said gap comprising the space between said light source and said detector.

10. An apparatus as claimed in claim 7, wherein said cell further comprises a light source supplying said wavelengths to said cell and a detector receiving wavelengths not absorbed by said mixture.

11. an apparatus as claimed in claim 10, further comprising a reflective surface in said cell, said reflective surface reflecting said wavelength from said light source to said detector.

12. An apparatus as claimed in claim 11, wherein said reflective surface has a mat finish.

13. An apparatus as claimed in claim 10, further comprising a light transmitting means communicating said light source and detector means to said cell.

14. An apparatus as claimed in claim 13, wherein said light transmitting means is a fiber optic.

**Patentansprüche**

1. Ein Verfahren zur Bestimmung der Anteile eines Kraftstoffes in einer Kraftstoffmischung über einen weiten Temperaturbereich, das die Schritte des Sendens eines Lichtstrahles mit zwei oder mehr Wellenlängen durch diese Kraftstoffmischung, das Messen der relativen Extinktion dieser Kraftstoffmischung bei jeder dieser Wellenlängen, das Messen der Temperatur dieser Kraftstoffmischung, wobei sich die zur Messung der Temperatur verwendete Vorrichtung von den zur Messung der relativen Extinktion verwendeten Vorrichtungen unterscheidet, sowie das Analysieren dieser relativen Extinktion und der Temperatur umfaßt, um den Anteil dieses Kraftstoffes in dieser Kraftstoffmischung zu bestimmen.

2. Ein Verfahren nach Anspruch 1, worin diese Wellenlängen eine Referenzwellenlänge und eine Wellenlänge für das Absorptionsmaximum umfassen, wobei diese Referenzwellenlänge so ausgewählt wird, daß sie von dieser Mischung schwach absorbiert wird, und diese Wellenlänge des Absorptionsmaximums so ausgewählt wird, daß sie von dieser Mischung stark absorbiert wird.

3. Ein Verfahren nach irgendeinem der Ansprüche 1 bis 2, das ferner das Einbringen dieser Mischung in eine Zelle mit einem Spalt vor dem Schritt des Sendens dieses Lichtstrahles umfaßt.

4. Ein Verfahren nach Anspruch 3, worin diese Zelle einen festen Spalt einer solchen Größe besitzt, daß eine nahezu lineare Absorption bereitgestellt wird.

**5.** Ein Verfahren nach Anspruch 4, daß ferner das Leiten dieser Mischung durch diese Zelle und das Messen der Extinktion dieser Mischung in diesem Spalt umfaßt.

**6.** Ein Verfahren nach Anspruch 2, worin diese Wellenlänge des Maximums zwischen 1400 und 1650 nm und diese Referenzwellenlänge zwischen 1100 und 1350 nm liegt.

**7.** Eine Vorrichtung zur Bestimmung der Anteile eines Kraftstoffes in einer Kraftstoffmischung über einen weiten Temperaturbereich, die eine diese Mischung enthaltende Zelle (100), eine Lichtquelle (90), die Licht zu dieser Mischung sendet, das mindestens eine erste und eine zweite Wellenlänge einschließt, Vorrichtungen (180, 190) zur Messung der relativen Extinktion dieser Mischung bei dieser ersten und dieser zweiten Wellenlänge, eine Vorrichtung (124) zur Messung der Temperatur dieser Mischung, wobei sich die zur Messung der Temperatur verwendete Vorrichtung (124) von den zur Messung der relativen Extinktion verwendeten Vorrichtungen (180, 190) unterscheidet, sowie eine Vorrichtung zur Analyse dieser relativen Extinktion und der Temperatur umfaßt, um den (die) Anteil(e) dieses Kraftstoffes in dieser Mischung zu bestimmen.

**8.** Eine Vorrichtung nach Anspruch 7, worin diese Zelle ferner einen Spalt umfaßt und diese relative Extinktion an diesem Spalt gemessen wird.

**9.** Eine Vorrichtung nach Anspruch 8, worin diese Zelle ferner eine Lichtquelle für die Zuführung dieser Wellenlängen und einen Sensor für die Messung dieser Wellenlängen umfaßt, wobei dieser Spalt durch den Abstand zwischen dieser Lichtquelle und diesem Sensor gebildet wird.

**10.** Eine Vorrichtung nach Anspruch 7, worin diese Zelle ferner eine Lichtquelle umfaßt, die dieser Zelle Wellenlängen zuführt, sowie einen Sensor, der die Wellenlängen empfängt, die nicht von dieser Mischung absorbiert werden.

**11.** Eine Vorrichtung nach Anspruch 10, die ferner eine reflektierende Oberfläche in dieser Zelle umfaßt, wobei diese reflektierende Oberfläche diese Wellenlänge von dieser Lichtquelle zu diesem Sensor reflektiert.

**12.** Eine Vorrichtung nach Anspruch 11, worin diese reflektierende Oberfläche matt poliert ist.

**13.** Eine Vorrichtung nach Anspruch 10, die ferner eine Vorrichtung zur Übertragung von Licht umfaßt, die diese Lichtquelle und diese Sensorvorrichtung mit dieser Zelle verbindet.

**14.** Eine Vorrichtung nach Anspruch 13, worin diese Vorrichtung für die Übertragung von Licht eine Fiberoptik ist.

## Revendications

**1.** Procédé de détermination de la proportion d'un carburant dans un mélange de carburants dans une large plage de températures comprenant les étapes consistant en l'émission d'un faisceau lumineux à travers ledit mélange de carburants à deux ou plusieurs longueurs d'onde, la mesure de l'absorbance relative dudit mélange de carburants à chacune desdites longueurs d'onde, la mesure de la température dudit mélange de carburants, le moyen utilisé pour mesurer la température étant distinct du moyen utilisé pour mesurer l'absorbance relative, et l'analyse de ladite absorbance relative et de la température en vue de déterminer la proportion dudit carburant dans ledit mélange de carburants.

**2.** Procédé selon la revendication 1, dans lequel lesdites longueurs d'onde comprennent une longueur d'onde de référence et une longueur d'onde à absorbance maximale, ladite longueur d'onde de référence étant choisie de manière à être faiblement absorbée par ledit mélange et ladite longueur d'onde à absorbance maximale étant choisie de manière à être fortement absorbée par ledit mélange.

**3.** Procédé selon l'une quelconque des revendications 1 à 2, comprenant en outre l'introduction dudit mélange dans une cuve à espacement, préalablement à l'étape d'émission dudit faisceau lumineux.

**4.** Procédé selon la revendication 3, dans lequel ladite cuve présente un espacement fixe calibré pour obtenir une absorbance quasi linéaire.

**5.** Procédé selon la revendication 4, comprenant en outre le passage dudit mélange à travers ladite cuve et la mesure de l'absorbance dudit mélange au niveau dudit espacement.

6. Procédé selon la revendication 2, dans lequel ladite longueur d'onde à absorbance maximale est comprise entre 1400 et 1650 nm et ladite longueur d'onde de référence est comprise entre 1100 et 1350 nm.

7. Appareil pour déterminer la proportion de carburant dans un mélange de carburants dans une large plage de températures comprenant une cuve (100) contenant ledit mélange, une source lumineuse (90) émettant de la lumière en direction dudit mélange comprenant au moins une première et une deuxième longueur d'onde, des moyens (180, 190) de mesure de l'absorbance relative dudit mélange à ladite première et deuxième longueur d'onde, des moyens (124) de mesure de la température dudit mélange, le moyen (124) de mesure de la température étant distinct des moyens (180, 190) de mesure de l'absorbance relative, et des moyens d'analyse de ladite absorbance relative et de la température afin de déterminer la ou les proportions dudit carburant dans ledit mélange.

8. Appareil selon la revendication 7, dans lequel ladite cuve comprend en outre un espacement et ladite absorbance relative est mesurée au niveau dudit espacement.

9. Appareil selon la revendication 8, dans lequel ladite cuve comprend en outre une source lumineuse pour générer lesdites longueurs d'onde et un détecteur pour mesurer lesdites longueurs d'onde, ledit espacement comprenant l'intervalle entre ladite source lumineuse et ledit détecteur.

10. Appareil selon la revendication 7, dans lequel ladite cuve comprend en outre une source lumineuse projetant lesdites longueurs d'onde vers ladite cuve et un détecteur recevant les longueurs d'onde non absorbées par ledit mélange.

11. Appareil selon la revendication 10, comprenant en outre une surface réfléchissante dans ladite cuve, ladite surface réfléchissante réfléchissant ladite longueur d'onde en provenance de ladite source lumineuse vers ledit détecteur.

12. Appareil selon la revendication 11, dans lequel ladite surface réfléchissante présente une couche de finition matte.

13. Appareil selon la revendication 10, comprenant en outre un moyen de transmission de lumière reliant ladite source lumineuse et moyen de détection à ladite cuve.

14. Appareil selon la revendication 13, dans lequel ledit moyen de transmission de lumière est une fibre optique.

NIR SPECTRUM OF GASOLINE

FIG.1A

NIR SPECTRUM OF METHANOL

FIG.1B

NIR SPECTRUM OF NON-EMULSIFIED METHANOL / GASOLINE MIXTURES

FIG.2

NIR SPECTRUM OF EMULSION BY ADDITION OF WATER FOR M 35

FIG.3

14

NIR SPECTRUM OF EMULSION BY TEMPERATURE FOR M 35

FIG.4

FIG.5

180
200
LOG RATIO AMPLIFIER

220
DIFFERENCE AMPLIFIER

230
AMPLIFIER

300

190
LOG RATIO AMPLIFIER
210

240
VOLTAGE - TO - FREQUENCY CONVERTER

124
TEMPERATURE DIODE

270
VOLTAGE AMPLIFIER

250
PULSE-WIDTH GENERATOR

122
ENGINE CONTROL COMPUTER

FIG.6

800
EMULSION OUTPUT

820

830

810

TO MIXER

FIG.14

FIG.7A

FIG.7B

EP 0 494 734 B1

1600 nm INPUT

N

L

1300 nm INPUT

C

D

TEMP. DIODE

S

T

230

270

METHANOL FREQ OUTPUT

U

V

252

FIG.8

FIG.9

FIG.10

EMULSION OUTPUT

800

ANALOG OUTPUT

R

S

METHANOL FREQ OUTPUT

U

V

TEMP. OUTPUT

X

Y

FIG.11

1300nm INPUT

C

D

1600nm INPUT

F

H

K

TEMP. DIODE

M

N

NIR SPECTRUM OF WATER, METHANOL & ETHANOL

FIG.12

FIG.13

NIR SPECTRUM OF THE AROMATIC FEATURE IN COMMERCIAL GASOLINE